(19) Europäisches Patentamt
European Patent Office
Office européen des brevets

(11) EP 1 957 980 B1

(12) # EUROPÄISCHE PATENTSCHRIFT

(45) Veröffentlichungstag und Bekanntmachung des Hinweises auf die Patenterteilung:
**06.01.2010 Patentblatt 2010/01**

(21) Anmeldenummer: **06828568.3**

(22) Anmeldetag: **29.11.2006**

(51) Int Cl.:
***G01N 33/543*** *(2006.01)*

(86) Internationale Anmeldenummer:
**PCT/DE2006/002107**

(87) Internationale Veröffentlichungsnummer:
**WO 2007/062628 (07.06.2007 Gazette 2007/23)**

(54) **IMMUNOASSAY ZUR SIMULTANEN IMMUNCHEMISCHEN BESTIMMUNG EINES ANALYTEN (ANTIGEN) UND EINES GEGEN DEN ANALYTEN GERICHTETEN THERAPIEANTIKÖRPERS IN PROBEN (RECOVERY IMMUNOASSAY)**

IMMUNOASSAY FOR THE SIMULTANEOUS IMMUNOCHEMICAL DETERMINATION OF AN ANALYTE (ANTIGEN) AND A TREATMENT ANTIBODY TARGETING THE ANALYTE IN SAMPLES (RECOVERY IMMUNOASSAY)

DOSAGE IMMUNOLOGIQUE POUR ANALYSER SIMULTANEMENT PAR IMMUNOCHIMIE UN ANALYTE (ANTIGENE) ET UN ANTICORPS THERAPEUTIQUE DIRIGE CONTRE CET ANALYTE DANS DES ECHANTILLONS (DOSAGE IMMUNOLOGIQUE FAISANT INTERVENIR LA RECUPERATION)

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.12.2005 DE 102005057920**

(43) Veröffentlichungstag der Anmeldung:
**20.08.2008 Patentblatt 2008/34**

(73) Patentinhaber: **Strohner, Pavel**
**13187 Berlin (DE)**

(72) Erfinder: **Strohner, Pavel**
**13187 Berlin (DE)**

(74) Vertreter: **Baumbach, Friedrich et al**
**Patentanwalt**
**Robert-Rössle-Strasse 10**
**13125 Berlin (DE)**

(56) Entgegenhaltungen:
**EP-B1- 0 850 416    WO-A-93/10451**
**US-B1- 6 174 688**

- **HAMILTON ET AL: "Immunological methods for quantifying free and total serum IgE levels in allergy patients receiving Omalizumab (Xolair) therapy" JOURNAL OF IMMUNOLOGICAL METHODS, ELSEVIER SCIENCE PUBLISHERS B.V.,AMSTERDAM, NL, Bd. 303, Nr. 1-2, August 2005 (2005-08), Seiten 81-91, XP005037172 ISSN: 0022-1759 in der Anmeldung erwähnt**
- **BEUM P V ET AL: "Three new assays for rituximab based on its immunological activity or antigenic properties: analyses of sera and plasmas of RTX-treated patients with chronic lymphocytic leukemia and other B cell lymphomas" JOURNAL OF IMMUNOLOGICAL METHODS, ELSEVIER SCIENCE PUBLISHERS B.V.,AMSTERDAM, NL, Bd. 289, Nr. 1-2, Juni 2004 (2004-06), Seiten 97-109, XP004520883 ISSN: 0022-1759**
- **PATTON ET AL: "An acid dissociation bridging ELISA for detection of antibodies directed against therapeutic proteins in the presence of antigen" JOURNAL OF IMMUNOLOGICAL METHODS, ELSEVIER SCIENCE PUBLISHERS B.V.,AMSTERDAM, NL, Bd. 304, Nr. 1-2, September 2005 (2005-09), Seiten 189-195, XP005065150 ISSN: 0022-1759**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- **PAGANELLI R ET AL: "STUDIES ON THE IN VITRO EFFECTS OF AUTO-ANTI-IGE. INHIBITION OF TOTAL AND SPECIFIC SERUM IGE DETECTION BY A HUMAN IGG AUTOANTIBODY TO IGE" JOURNAL OF CLINICAL AND LABORATORY IMMUNOLOGY, TREVIOT-KIMPTON PUBLICATIONS, LONDON, GB, Bd. 26, Nr. 3, Juli 1988 (1988-07), Seiten 153-157, XP008078693 ISSN: 0141-2760**

Bemerkungen:

Die Akte enthält technische Angaben, die nach dem Eingang der Anmeldung eingereicht wurden und die nicht in dieser Patentschrift enthalten sind.

## Beschreibung

Die Erfindung bezieht sich auf einen Immunoassay zur gleichzeitigen immunchemischen Bestimmung eines Analyten (Antigen) und eines gegen den Analyten gerichteten Therapieantikörpers.
Anwendungsgebiete der Erfindung sind die medizinische Diagnostik, die Therapiekontrolle und die pharmakologische Forschung.
Bisher bekannt ist folgender Stand der Technik:

A) Zum Immunoassay
Der Immunoassay ist eine sehr verbreitete Technik zur Bestimmung unbekannter Konzentrationen analytisch oder diagnostisch relevanter Stoffe (Analyt) in Probenmaterial wie Serum, Plasma, Gewebsproben, Rückstandspräparationen, Überstände von Zellkulturen und ähnlichem.
Die Immunoassays (Radioimmunoassays(RIA), Enzymimmunoassays (EIA), Lumineszenzimmunoassays(LIA) und damit verwandte Assayvarianten) basieren darauf, dass eine Analytkonzentration als Standard vorgeben wird und für diese nach Reaktion mit einem Antikörper unter Einbeziehung einer markierten Verbindung (eines markierten Antigens oder Antikörpers) die Response des gebundenen markierten Antigens oder Antikörpers (Impulsrate, Extinktion, Relative Light Units) bestimmt wird. Der Zusammenhang zwischen Response und Analytkonzentration des Standards wird in Form einer Standardkurve mittels mathematischer Eichfunktion oder graphisch beschrieben.
Die Analytkonzentration unbekannter Proben wird unter Verwendung der nach der Analytkonzentration umgeformten Eichfunktion aus der Response der unbekannten Probe errechnet oder wird an der graphisch erstellten Standardkurve abgelesen. Bei der Eichung mit Hilfe einer Standardkurve werden gewöhnlich zur Vermeidung der Beeinflussung der Messung durch Fremdstoffe besondere Vorbehandlungen der Proben (z.B. Extraktionen, Zugabe von Zusatzstoffen) oder der Assaykomponenten (Einbringung der Standards in 0-Seren, Proteinzusätze u.a.m.) vorgenommen. Mit Wiederfindeexperimenten wird die Richtigkeit der Bestimmungen an den so modifizierten Assaykomponenten oder Proben geprüft.
Softwarepakete an den Messgeräten (Gamma-Counter, Extinktions-Reader, Lumineszenz-Messgeräte), die die Immunoassay-Auswertung übernehmen, sind Standardausstattung von Immunoassay-Forschungs- und Routinelabors geworden. Darüber hinaus sind Immunoassay-Laborautomaten verschiedenster Hersteller auf dem Markt, die sowohl die Abarbeitung eines Immunoassays als auch seine Messung und Auswertung in einem Automaten vereinen. Allen diesen Auswerte-Software-Paketen und Labor-Automaten liegt o.g. Eichverfahren zugrunde, wenn auch heute, auf Grund der besseren Beherrschung der Assaytechnik, gespeicherte Eichfunktionen für mehrere Assayläufe oder auf zwei Standardkonzentrationen reduzierte Eichgeraden üblich geworden sind.

B) Therapieantikörper
Es sind in den letzten Jahren eine Reihe von Therapieantikörpern entwickelt worden bzw. sind noch in der Entwicklung. Diese sind vorwiegend auf die Behandlung von Entzündungen, Autoimmunerkrankungen und Krebs ausgerichtet. Viele dieser Therapieantikörper sind zwar ursprünglich als monoklonale Antikörper erzeugt, die meisten von ihnen sind jedoch zur Vermeidung der Immunabwehr des Patienten gegen den Therapieantikörper gentechnisch in humane Antikörper umgewandelt worden. Einige von ihnen sind Chimeren von humanen und monoklonalen Antikörpern oder sogar nur monoklonale Antikörper.
Tabelle 1 zeigt eine Auswahl von Therapieantikörpern, die bereits als Arzneimittel zugelassen sind bzw. sich in der Zulassung befinden:

Diese Therapieantikörper sind gegen krankheitsspezifische humane Proteine gerichtet. Zwar sind auf der Grundlage von Sandwich-Immunoassays die betroffenen krankheitsspezifischen humanen Proteine als Antigen bestimmbar, doch wird diese Bestimmung durch die Therapieantikörper verfälscht und es sind spezifische Assaykonzepte erforderlich (siehe Hamilton,R.G. et.al.), um freies und Gesamtantigen zu bestimmen.

Tabelle 1

| **Name des Therapieantikörpers** | **Entwickler; Produzent** | **Antikörpertyp** | **Antigen; Indikation** |
|---|---|---|---|
| Omalizumab (Xolair) | Genentech; Novartis; Tanox | humanisiert | IgE; Allergisches Asthma |
| Infliximab (Remicade) | Centocor | Chimere | TNF-$\alpha$; Rheuma, Morbus Crohn |
| Adalimumab (Humira) | Cambridge Antibody Technology;Abbott | humanisiert | TNF-$\alpha$; Rheuma, |

(fortgesetzt)

| Name des Therapieantikörpers | Entwickler; Produzent | Antikörpertyp | Antigen; Indikation |
|---|---|---|---|
| Muromomab-CD3 | Ortho Biotech; Johnson&Johnson | monoklonal | CD3; Verhinderung der Abstoßung von Organtransplantaten |
| Daclizumab (Zenapax) | Protein Design Labs | humanisiert | CD25; Verhinderung der Abstoßung von Nierentransplantaten |
| Basiliximab (Simulect) | Novartis | Chimere | CD25; Verhinderung der Abstoßung von Nierentransplantaten |
| Raptiva | Genentech; Serono,Xoma | humanisiert | BLA[a], CD11a; Psoriasis |
| Natalizumab (Antegren) | Elan, Biogen | humanisiert | VLA-4β1[b]; Morbus Crohn, Multiple Sklerose |
| CDP-870 | Celltech; Pfizer | humanisiert | TNF-α; Rheuma, Morbus Crohn |
| Trastuzumab (Herceptin) | Genentech | humanisiert | HER-2/neu (p183[neu]); Brustkrebs, Lungenkrebs, Pancreas Krebs |
| Daclizumab (Zenapax) | Protein Design Labs | Chimere | IL-2R; Leukemie |
| Endrecolomab (Panorex) | Johnson&Johnson | humanisiert | 17-A1; Colorectalcarcinom |

In der Europäischen Patentschrift EP 0850416B1 vom 01.08.2001 ist mit dem Verfahren zur zweidimensionalen Bestimmung von Proben im Immunoassay eine Methode beschrieben, die den Einfluss von kreuzreaktiven Stoffen und Matrixeffekten analysieren kann und die Antigenkonzentration entsprechend berichtigt.

Bisher gibt es noch kein Verfahren, das die gleichzeitige richtige Bestimmung eines Antigens und des gegen das Antigen gerichteten Therapieantikörpers ermöglicht.

Der Erfindung liegt die Aufgabe zugrunde, ein einfaches und schnell durchzuführendes Verfahren zu entwickeln, mit dem ein Antigen und ein dagegen gerichteter Therapieantikörper gleichzeitig in einer Lösung bestimmt werden können. Es soll vorzugsweise für eine Therapiekontrolle einsetzbar sein. Die Aufgabe wird durch ein Verfahren zur Verwendung eines Immunoassays gemäß Anspruch 1 gelöst.
Der Immunoassay umfasst:

- einen an einer Oberfläche gebundenen gegebenenfalls markierten Fängerantikörper
- einen markierten Therapieantikörper, oder ein markierter Antikörper, der das gleiche Bindungsepitop wie der Therapieantikörper aufweist ein Antigen, das mit dem Fängerantikörper und dem Therapieantikörper an verschiedenen Epitopen bindet und dadurch einen immunochemischen Sandwich bildet
- eine Lösung des unmarkierten Therapieantikörpers bekannter Konzentration und
- eine Antigenlösung zur Aufstockung der Proben.

**Grundprinzip der Erfindung**

Der Assay geht von einem gewöhnlichen Sandwich-Immunoassay aus.
In einem richtig bestimmenden Sandwich-Immunoassay, bei dem der Therapieantikörper oder ein Antikörper, der am gleichen Bindungsepitop bindet, in markierter Form als Nachweisantikörper oder Fängerantikörper verwendet wird, bewirkt die Anwesenheit des Therapieantikörpers in Proben eine systematische Verringerung der Wiederfindung des zu bestimmenden Antigen, die mit der Konzentration des Therapieantikörpers korreliert. Diese Korrelation kann zur Bestimmung der unbekannten Konzentration des Therapieantikörpers und zur Bestimmung der freien unbekannten Konzentration des Therapieantikörpers und zur Bestimmung der freien und totalen Antigenkonzentration genutzt werden. Aus diesem Grunde kann dieses Immunoassay auch kurz Wiederfinde-Immunoassay oder Recovery-Immunoassay genannt werden.

**Vorgehensweise:**

Es wird für das nachzuweisende Antigen ein gewöhnlicher Sandwichimmunoassay entwickelt. Dieser Sandwich-Immunoassay hat folgenden Aufbau:

a) Ein Fängerantikörper, an der Mikrotiterplatten oder Biochipoberfläche immobilisiert.
b) Der Analyt ist ein Protein oder ein anderes Biopolymer, das mit einem Bindungsepitop am Fängerantikörper bindet, der als Standard oder Probe im Immunoassay gemessen wird.
c) Der Nachweisantikörper ist der Therapieantikörper, der radioaktiv oder nichtradioaktiv markiert ist und an einem anderen Bindungsepitop als der Fängerantikörper den Analyten bindet. Als nichtradioaktive Markierungen können alle im Immunoassay üblichen Verfahren (mit Peroxydase, mit alkalischer Phosphatase, mit Luminiszenz- oder Fluoreszenzlabeln) benutzt werden.

Es werden nun Sandwich-Immunoassays ohne und mit Zusatz von 2 bis 5 verschiedenen Antikörperkonzentrationen des nichtmarkierten humanisierten Therapieantikörpers durchgeführt. Dabei ist für den Sandwich-Immunoassay ohne den nichtmarkierten humanisierten Therapieantikörpers wesentlich, dass der Assay in den zu untersuchenden Proben die Antigenkonzentrationen richtig wiedergibt.
Die Messwerte der 2 bis 6 Immunoassays werden nun daraufhin analysiert, in welchem Maße der Zusatz der humanisierten Therapieantikörper eine Reduktion der Messwerte und damit der Wiederfindung der Antigenkonzentration in Standardkurven bewirkt. Der Zusammenhang zwischen Reduktion der Messwerte = Reduktion der rekalkulierten Antigenkonzentration (Standardkonzentration) und Zusatz der humanisierten Therapieantikörper wird mit Hilfe einer statistischen Auswertung in eine geeignete mathematische Funktion zusammengefasst. Es wird mit dieser Funktion (Wiederfindefunktion) ein Zusammenhang zwischen der Wiederfindung des Antigens im Immunassay und der Konzentration des Therapieantikörpers hergestellt. Bei starkem Überschuß von Fängerantikörper und markierten Therapieantikörper ist die Beziehung zwischen dem Kehrwert der Wiederfindung des Antigens im Immunassay und der Konzentration des Therapieantikörpers eine einfache lineare Funktion. Voraussetzung für die Gültigkeit dieser Funktion ist, dass Verfälschungen der Messwerte durch Matrixeffekte in den Proben ausgeschlossen werden. Aus diesem Grunde müssen die Reaktionsmedien der Standardlösungen und der Proben vergleichbar sein.
Unbekannte Proben werden mit 2-3 bekannten Antigenkonzentrationen (Standards) aufgestockt. Es wird nun im Immunoassay festgestellt, wie die Wiederfindung der aufgestockten, bekannten Antigenkonzentrationen in der unbekannten Probe ist. Falls die Wiederfindung um die 100 % beträgt, ist kein Therapieantikörper in der Probe enthalten. Wenn die Wiederfindung kleiner ist, kann mit der o.g. Wiederfindefunktion indirekt auf die Konzentration des Therapieantikörpers in den Proben und auf die tatsächliche totale Konzentration des Antigens geschlossen werden.
Es stehen nur sehr aufwändige Verfahren zur Bestimmung des gesamten und freien IgE zur Verfügung. In der Publikation Hamilton R.G. et al. sind 2 gesonderte Elisas zur Bestimmung erforderlich, wobei eine Bestimmung der wirksamen Antikörperkonzentration nicht erfolgt.
Mit Hilfe des erfindungsgemäßen Verfahrens ist eine Therapiekontrolle beispielsweise bei dem Einsatz von Omalizumab bei allergischem Asthma und anderen Autoimmunerkrankungen sehr einfach durchzuführen.
Nachfolgende 2 Beispiele sollen das Verfahren näher erläutern:

**Beispiel 1:**

Auswirkung des Zusatzes des Therapieantikörpers Omalizumab (Xolair=E-25) auf den IgE-Enzymimmunoassay auf Streptavidin-beschichtete Mikrotiterplatten im Puffer

Material:

1. Streptavidin beschichtete Mikrotiterplatten, Beschichtungsverfahren der BioTeZ Berlin-Buch GmbH für Maximale Biotin-Bindungskapazität (MC-Beschichtung)
2. Fängerantikörper zur Immobilisierung an den Streptavidin beschichteten Mikrotiterplatten biotinylierter Antikörper B 216 / 290702; anti-human IgE, mAb E-411
3. IgE-Standard: IgE von OEM
4. POD-markierter Nachweisantikörper PD 08/110501 von Hum., mAk E25 markiert mit POD, Novartis;
5. Humanisierter monoklonaler Therapieantikörper Omalizumab(Xolair)=E-25 von Novartis
6. Beschichtungspuffer (PBS 0,05M, pH=7,2 mit 0,1% RSA)
7. Reaktionspuffer (PBS/ 0,33 % Casein + 0,0125 % TWEEN 20)

8. Waschlösung (0,9% NaCl/0,1% Tween)
9. Enzymsubstrat: Orthophenylendiamin (OPD)

In Tabelle 2 ist der Versuchsaufbau auf der Mikrotiterplatte dargestellt und in Tabelle 3 das Ergebnis des Versuchs.

Versuchsablauf:

1. Immobilisierung des Fängerantikörpers in allen Wells der Mikrotiterplatte. Zugabe vom 200 µl Fängerantikörperlösung (4 µg/ml). Inkubation über Nacht bei 4° C
2. Am nächsten Tag 2* Waschen im MTP-Washer mit Waschlösung
3. Herstellung der IgE-Standardreihe 0 / 3,125 / 6,25 / 12,5 / 25 / 50 / 100/ 200 ng IgE/ml gemäß Versuchsaufbau
4. Herstellung der E-25-Verdünnungen gemäß Versuchsaufbau 0 / 0,0625 / 0,125 / 0,25 / 0,5 / 1 µg/ml
5. Mischen je 50 µl 3. und 4. zu einer Ansatzlösung
6. Mischen der Ansatzlösung (100 µl) mit dem Nachweiskonjugat (PD08, 100 ng/ml, 100 µl)
7. Zugabe von 200 ml Mischung aus Ansatzlösung und Nachweiskonjugat zur Mikrotiterplatte
8. Inkubation der Mischung über 3 Stunden bei Raumtemperatur
9. 2* Waschen im MTP-Washer mit Waschlösung
10. Enzymnachweis mit OPD (14 mg OPD auf 20 ml Sörensenpuffer+ 80 µl 3% $H_2O_2$), Zugabe von 200 µl OPD-Lösung und Abstoppen der Enzymreaktion nach 30 min mit 50 µl $H_2SO_4$ [5M]

Tabelle 2: Versuchsaufbau auf der Mikrotiterplatte:

| | Alle Messungen als Duplikate | 1 | 2 | 3 | 4 | 5 | 6 | 7 | 8 | 9 | 10 | 11 | 12 |
|---|---|---|---|---|---|---|---|---|---|---|---|---|---|
| Zeile | E-25-Gehalt (µg/ml) ⇒ | 0 | 0 | 0,033 | 0,033 | 0,063 | 0,063 | 0,125 | 0,125 | 0,25 | 0,25 | 0,5 | 0,5 |
| Spalte | IgE-Konz. ng/ml ⇓ | | | | | | | | | | | | |
| A | 0 | | | | | | | | | | | | |
| B | 1,5625 | | | | | | | | | | | | |
| C | 3,125 | | | | | | | | | | | | |
| D | 6,25 | | | | | | | | | | | | |
| E | 12,5 | | | | | | | | | | | | |
| F | 25 | | | | | | | | | | | | |
| G | 50 | | | | | | | | | | | | |
| H | 100 | | | | | | | | | | | | |

Tabelle 3: Ergebnis des Versuchs Mittelwerte der Doppelbestimmungen als Extinktionen (O.D.: Optical Density)

| | E-25-Gehalt in ng /ml ⇒ | 0 | 31,250 | 62,5 | 125 | 250 | 500 |
|---|---|---|---|---|---|---|---|
| | IgE-Konz. ng/ml ⇓ | O.D. | O.D. | O.D. | O.D. | O.D. | O.D. |
| A | 0 | 0.070 | 0,064 | 0,054 | 0,054 | 0,052 | 0,064 |
| B | 1,563 | 0,154 | 0,149 | 0,143 | 0,112 | 0,102 | 0,104 |
| C | 3,125 | 0,201 | 0,192 | 0,169 | 0,145 | 0,127 | 0,119 |
| D | 6,25 | 0,277 | 0,266 | 0,256 | 0,186 | 0,156 | 0,146 |
| E | 12,5 | 0,493 | 0,501 | 0,434 | 0,365 | 0,290 | 0,244 |

(fortgesetzt)

| | E-25-Gehalt in ng /ml ⇒ | 0 | 31,250 | 62,5 | 125 | 250 | 500 |
|---|---|---|---|---|---|---|---|
| | IgE-Konz. ng/ml ⇓ | O.D. | O.D. | O.D. | O.D. | O.D. | O.D. |
| F | 25 | 0,984 | 0,879 | 0,805 | 0,707 | 0,585 | 0,382 |
| G | 50 | 1,482 | 1,257 | 1,198 | 0,948 | 0,751 | 0,587 |
| H | 100 | 2,378 | 2,285 | 2,216 | 1,927 | 1,432 | 1,134 |
| Abbildung 1. zeigt den IgE-ELISA in Abhängigkeit vom E-25-Zusatz. | | | | | | | |

Wenn man nun die Messergebnisse (O.D.) der IgE-Standardkurven bei unterschiedlichem E-25-Zusatz auf die IgE-Standardkurve ohne E-25-Zusatz bezieht, ergibt sich folgender sehr einfacher linearer Zusammenhang (Tabelle 4):

Tabelle 4:

| **E-25-Zusatz in µg/ml zu den IgE-Standardlösungen** | 0 | 0,03125 | 0,0625 | 0,125 | 0,25 | 0,5 |
|---|---|---|---|---|---|---|
| Bestimmtheitsmaß des linearen Zusammenhangs zu der Standardreihe ohne E-25-Zusatz | 1,000 | 0,994 | 0,993 | 0,984 | 0,990 | 0,981 |
| Wiederfindung der IgE-Standards in dem Messwerten (O.D.) | 100,00% | 93,05% | 90,32% | 77,62% | 57,77% | 44,06% |

Es zeigt sich somit, dass alle Messwerte der IgE-Standardkurven mit E-25-Zusatz stark linear mit der IgE-Standardkurve ohne E-25-Zusatz korreliert sind. Die Wiederfindung der im ELISA eingesetzten IgE-Standardlösungen wird mit zunehmenden E-25-Gehalt systematisch verringert. Es ergibt sich folgender einfacher Zusammenhang zwischen E-25-Gehalt und Wiederfindung des IgE-Standards:

$$1/\text{Wiederfindung} = 1 + 2{,}626 * \text{E-25-Gehalt} \quad \text{(Formel 1)}$$

Das Bestimmtheitsmaß des lineare Zusammenhangs des reziproken Wertes der Wiederfindung zu der E-25-Konzentration betrug 0,9907.

Der Immunoassay ist jedoch für die Bestimmung unbekannter Antigenkonzentrationen gedacht. Aus diesem Grunde werden für Standardverdünnungen bekannter Antigenkonzentrationen die Messwerte im Immunoassay als sogenannte Standardkurve ermittelt. Der Zusammenhang zwischen den bekannten Antigenkonzentrationen und den Messwerten wird für den Immunoassay üblichen mathematischen Funktionen mit Hilfe der Regression erfasst und zur Berechnung der Antigenkonzentrationen unbekannter Proben aus ihren Messwerten im Immunoassay genutzt.

$$\text{Logit}(E) = \ln((E\text{-NSB})/(E_{max}\text{-}E\text{+NSB})) = a + b^* \ln(X) \quad \text{(Formel 2)}$$

In: natürlicher Logarithmus
E: Extinktion (Messwert im Enzymimmunoassay) NSB: unspezifische Bindung
Emax: Maximale Extinktion
a,; b: Parameter der Regressionsfunktion

Es ergab sich durch lineare Regression folgende Auswertefunktion der Standardkurve: Logit(E) = 0,892*In (IgE)-5,30775 (Formel 3)

Das Bestimmtheitsmaß für den linearen Zusammenhang war 99,292 %. Die Auswertefunktion gibt den Zusammenhang zwischen Messwerten und Igel-Konzentration zu 99,3 % richtig wieder; nur 0,7 % der Messwerte weichen davon zufällig ab.

Die aus der Auswertefunktion abgeleitete Umkehrfunktion:

$$\ln(IgE) = (5{,}30775 + Logit(E))/0{,}892 \text{ (Formel 4)}$$

kann mit sehr geringem Fehler zur Berechnung unbekannter Proben aus den Messwerten (hier Extinktionen) benutzt werden.

Mit Hilfe dieser Umkehrfunktion wurden nun aus den Messwerten (Extinktionen) die IgE-Konzentrationen in den Standardkurven mit E-25-Zusatz ermittelt (Tabelle 5):

Tabelle 5:

| **E-25-Gehalt μg/mL** | **0** | **0,03125** | **0,0625** | **0,125** | **0,25** | **0,5** |
|---|---|---|---|---|---|---|
| **IgE ng/ml** | IgE, rekalk. | IgE, rekalk, | IgE, rekalk. | IgE, rekalk. | IgE, rekalk. | IgE, rekalk. |
| **1,56** | 1,81 | 1,69 | 1,56 | 0,82 | 0,62 | 0,66 |
| **3,13** | 3,02 | 2,77 | 2,20 | 1,61 | 1,17 | 0,99 |
| **6,25** | 5,09 | 4,78 | 4,49 | 2,63 | 1,87 | 1,62 |
| **12,50** | 11,61 | 11,87 | 9,74 | 7,63 | 5,46 | 4,17 |
| **25,00** | 29,25 | 25,19 | 22,41 | 18,85 | 14,64 | 8,14 |
| **50,00** | 50,74 | 40,55 | 38,03 | 27,82 | 20,43 | 14,72 |
| **100,00** | 99,60 | 93,84 | 89,64 | 73,20 | 48,41 | 35,32 |
| **Bestimmtheitsmaß** | | 0,994 | 0,994 | 0,985 | 0,988 | 0,993 |
| **Wiederfindung** | | 92,23% | 88,50% | 71,91% | 47,86% | 34,74% |

IgE-rekalk.: Durch die Umkehrfunktion der Auswertefunktion bestimmten IgE-Konzentrationen.

Wie bei den Messwerten ergab sich erwartungsgemäß auch hier eine systematische Abnahme der gemessenen IgE-Konzentrationen in Abhängigkeit vom E-25-Gehalt in den Standardproben. Es zeigte sich wie bei den Messwerten ein einfacher lineare Zusammenhang zwischen den IgE-Werten ohne und mit E-25-Zusatz (Alle Bestimmtheitsmaße waren über 98,5 %).

Der Zusammenhang E-25-Gehalt und Wiederfindung der IgE-Konzentrationen in den bekannten Standardkonzentrationen konnte durch eine einfache Funktion wiedergegeben werden (Abbildung 2), wobei 1/WF = 0,9606 + 3,932*X (Formel 5);

WF: Wiederfindung; X: E-25-Gehalt in μg/ml;

Das Bestimmtheitsmaß für diese Funktion war 98,74 %.

Mit der Umkehrfunktion dieser Funktion kann man aus den Wiederfindewerten somit den E-25-Gehalt in den Proben abschätzen.

In diesem Beispiel 1 sollte nur das Grundprinzip der Bestimmung der Konzentrationen des Therapieantikörpers E-25-(Xolair) in bekannten Proben gezeigt werden. Auch wurden die Immunoassays nur in Pufferlösungen ausgeführt.

Im Beispiel 2 soll nunmehr auch gezeigt werden, dass nach gleichem Verfahren auch in unbekannten Serumproben die Bestimmung des E-25-Gehalts durch einfache Aufstockungsexperimente im Immunoassay möglich ist. Um die Vergleichbarkeit der Reaktionslösungen von Standards und unbekannten Serumproben zu sichern, wurden durch Immunaffinitätschromatographie IgE-freie Seren erzeugt und in diesen die IgE-Standards im Immunoassay angesetzt.

**Beispiel 2:**

Auswirkung des Zusatzes des Therapieantikörpers Omalizumab (Xolair=E-25) auf die Wiederfindung unbekannter Serumproben im IgE-Enzymimmunoassay auf Streptavidin-beschichtete Mikrotiterplatten und Bestimmung des E-25-Gehalts in unbekannten Serumsproben, die mit E-25 versetzt wurden.

Material:

10. Streptavidin beschichtete Mikrotiterplatten, Beschichtungsverfahren der BioTeZ Berlin-Buch GmbH für Maximale Biotin-Bindungskapazität (MC-Beschichtung)

11. Fängerantikörper zur Immobilisierung an den Streptavidin beschichteten Mikrotiterplatten biotinylierter Antikörper B 216/290702; anti-human IgE, mAb E-411
12. IgE-Standard: IgE von OEM
13. POD-markierter Nachweisantikörper PD 08/110501 von Hum., mAk E25 markiert mit POD, Novartis;
14. Humanisierter monoklonaler Therapieantikörper Omalizumab(Xolair)=E-25 von Novartis
15. Beschichtungspuffer (PBS 0,05M, pH=7,2 mit 0,1% RSA)
16. Reaktionsmedium Standardkurven(1:10-verdünntes IgE-freies Serum in PBS/ 0,33 % Casein + 0,0125 % TWEEN 20)
17. Proben 1:10 verdünnt in PBS/ 0,33 % Casein + 0,0125 % TWEEN 20
18. Waschlösung (0,9% NaCl/0,1 % Tween)
19. Enzymsubstrat: Orthophenylendiamin (OPD)

In Tabelle 6 und 7 ist der Versuchsaufbau auf der Mikrotiterplatte, (1. und 2. Hälfte) dargestellt und in Tabelle 8 das Ergebnis des Immunoassays.

Tabelle 6: Versuchsaufbau auf der Mikrotiterplatte, 1.Hälfte

| | | Standardkurven | | | | | |
|---|---|---|---|---|---|---|---|
| | Alle Messungen als Duplikate | 1 | 2 | 3 | 4 | 5 | 6 |
| Zeile | E-25-Gehalt (μg/ml) ⇒ | 0 | 0 | 0,125 | 0,125 | 0,5 | 0,5 |
| Spalte | IgE-Konz. ng/ml ⇓ | | | | | | |
| A | 0 | | | | | | |
| B | 1,5625 | | | | | | |
| C | 3,125 | | | | | | |
| D | 6,25 | | | | | | |
| E | 12,5 | | | | | | |
| F | 25 | | | | | | |
| G | 50 | | | | | | |
| H | 100 | | | | | | |

Tabelle 7: Versuchsaufbau auf der Mikrotiterplatte, 2.Hälfte

| | | Unbekannte Proben | | | | | |
|---|---|---|---|---|---|---|---|
| | Alle Messungen als Duplikate | 7 | 8 | 9 | 10 | 11 | 12 |
| Zeile | Aufstockung der unbekannten Probe mit IgE in ng/ml ⇒ | 0 | 0 | 6,25 | 6,25 | 25 | 25 |
| Spalte | Unbekannte Proben | | | | | | |
| A | Serumprobe 3 | | | | | | |
| B | Serumprobe 5 | | | | | | |
| C | Serumprobe 6 | | | | | | |
| D | Serumprobe 7 | | | | | | |
| E | Serumprobe 3 + 0,031 μg/ml E-25 | | | | | | |
| F | Serumprobe 5 + 0,063 μg/m E-25 | | | | | | |
| G | Serumprobe 6 + 0,125μg/ml E-25 | | | | | | |
| H | Serumprobe 7 + 0,25 μg/ml E-25 | | | | | | |

Versuchsablauf:

1. Immobilisierung des Fängerantikörpers in allen Wells der Mikrotiterplatte. Zugabe vom 200 µl Fängerantikörperlösung (4 µg/ml). Inkubation über Nacht bei 4° C
2. Am nächsten Tag 2* Waschen im MTP-Washer mit Waschlösung
3. **Standardreihen**
Für Reihe A-H, Spalte 1-6: 0 / 3,125 / 6,25 / 12,5 / 25 / 50 /100/ 200 ng IgE/ml und 0 / 0,125 / 0,5 µg/ml E-25 in IgE-freiem Serum 1:10 verdünnt im Reaktionspuffer
4. **Proben**

| | | |
|---|---|---|
| Spalte 7-12, A-D | Serum 3, 5, 6, 7, 1:10-verdünnt in Puffer | Aufgestockt mit 0 / 6,25 / 25 ng/ml IgE |
| Spalte 7-12, E | Serum 3 1:10-verdünnt in Puffer + 0.031 µg/ml E-25 | Aufgestockt mit 0 / 6,25 / 25 ng/ml IgE |
| Spalte 7-12, F | Serum 5 1:10-verdünnt in Puffer + 0.063 µg/ml E-25 | Aufgestockt mit 0 / 6,25 / 25 ng/ml IgE. |
| Spalte 7-12, G | Serum 6 1:10-verdünnt in Puffer + 0.125 µg/ml E-25 | Aufgestockt mit 0 / 6,25 / 25 ng/ml IgE. |
| Spalte 7-12, H | Serum 7 1:10-verdünnt in Puffer + 0.25 µg/ml E-25 | Aufgestockt mit 0 / 6,25 / 25 ng/ml IgE |

5. Inkubation der Mischung über 3 Stunden bei Raumtemperatur
6. 2* Waschen im MTP-Washer mit Waschlösung
7. Enzymnachweis mit OPD (14 mg OPD auf 20 ml Sörensenpuffer+ 80 µl 3% $H_2O_2$), Zugabe von 200 µl OPD-Lösung und Abstoppen der Enzymreaktion nach 30 min mit50 µl $H_2SO_4$ [5M]

a) Standardkurven (Extinktionen)

Tabelle 8: Ergebnis des Immunoassays:

| | E25-Zusatz in µg/ml ⇒ | | |
|---|---|---|---|
| ng/ml IgE ⇓ | 0 | 0,125 | 0,5 |
| | O.D. | O.D. | O.D. |
| 0 | 0,045 | 0,039 | 0,050 |
| 1,5625 | 0,098 | 0,096 | 0,074 |
| 3,125 | 0,121 | 0,102 | 0,084 |
| 6,25 | 0,170 | 0,150 | 0,110 |
| 12,5 | 0,281 | 0,264 | 0,195 |
| 25 | 0,546 | 0,439 | 0,282 |
| 50 | 0,921 | 0,827 | 0,421 |
| 100 | 1,602 | 1,316 | 0,798 |
| Bestimmtheitsmaß | | 0,9968 | 0,9856 |
| Wiederfindung | 1 | 0,8283 | 0,4671 |
| O.D.: Messwerte des Enzymimmunoassays; Optical Density (Extinktion) | | | |

Es zeigten sich erwartungsgemäß auch in den Standardkurven im IgE-freie Serum mit Zusatz von E-25 systematische Erniedrigungen der Wiederfindung der Messwerte.

Der Zusammenhang zwischen den bekannten Antigenkonzentrationen und den Messwerten in der Standardkurve ohne E-25-Zusatz wurde auch hier mit den für den Immunoassay üblichen Logit-Log-Funktion mit Hilfe der Regression erfasst und zur Berechnung der mit E-25 versetzten IgE-Standards und zur Berechnung der IgE-Konzentrationen unbekannter Proben aus ihren Messwerten im Immunoassay genutzt.

Es ergab sich durch Regression

$$\text{Logit}(E) = 0{,}8984 \cdot \ln(\text{IgE}) - 5{,}9079. \quad \text{(Formel 6)}$$

Mit der Umkehrfunktion dieser Auswertefunktion (ln(IgE)= (5,9079+Logit(E))/0,8984) (Formel 7) wurden nun aus den Messwerten der mit E-25 versetzten Standards und der unbekannten Proben die IgE-Konzentrationen errechnet.
Es ergaben sich für die Standardkurven mit und ohne E-25-Zusatz folgende IgE-Konzentrationen (Tabelle 9):

Tabelle 9:

| **E-25-Zugabe in μg/ml** | **0** | **0,0625** | **0,25** |
|---|---|---|---|
| **Bekannte IgE-Standardkonz. in nglml** | Rekalkulierte IgE-Konz. in ng/ml | Rekalkulierte IgE-Konz. in ng/ml | Rekalkulierte IgE-Konz. in ng/ml |
| 0 | | | |
| 1,5625 | 1,91 | 1,82 | 0,88 |
| 3,125 | 2,95 | 2,08 | 1,30 |
| 6,25 | 5,29 | 4,29 | 2,42 |
| 12,5 | 11,02 | 10,11 | 6,54 |
| 25 | 26,48 | 20,02 | 11,10 |
| 50 | 51,62 | 44,98 | 18,93 |
| 100 | 101,94 | 81,97 | 43,01 |
| Wiederfindung des IgE-Standards | 102,709% | 83,349% | 42,050% |
| Bestimmtheitsmaß | 99,938% | 99,721% | 99,464% |

In den Standardkurven war eine systematische Abnahme der IgE-Konzentrationen im Enzymimmunoassay festzustellen. Der Zusammenhang zwischen E-25-Zugabe und IgE-Wiederfindung konnte mit folgender einfacher linearen Funktion wiedergegeben werden 8 (Abbildung 3), wobei Wf = 0,99 - 2,291 *X (Formel 8); Wf: Wiederfindung der IgE-Konzentration; X: E-25-Zusatz in μg/ml sind.
Bestimmtheitsmaß für diesen linearen Zusammenhang war 99,84 %.
Bei den unbekannten Serumproben ergaben sich folgende Ergebnisse im ELISA (Tabelle 10):

Tabelle 10: Anonyme Patientenseren ohne und mit E-25 Zusatz

| Aufstockung IgE(ng/ml) | E-25-Zusatz in µg/ml | 0,00 | 6,25 | 25,00 | Wiederfindung | Anfangswert | Bestimmtheitsmaß | Theoretis cher E-25-Gehalt µg/ml |
|---|---|---|---|---|---|---|---|---|
| **Serum 3** | | | | | | | | |
| Gemessene IgG in ng/ml | 0,000 | 10,54 | 20,97 | 38,60 | 108,04% | 12,114 | 0,982 | |
| Sollwert | | 12,11 | 16,79 | 35,54 | | | | |
| I Gemessene IgG in ng/ml | 0,031 | 7,46 | 12,86 | 32,84 | 102,68% | 7,021 | 0,998 | 0,01 |
| Sollwert | | 7,02 | 13,71 | 32,46 | | | | |
| **Serum 5** | | | | | | | | |
| Gemessene IgG in ng/ml | 0,000 | 14,70 | 23,92 | 41,06 | 102,19% | 15,915 | 0,988 | |
| Sollwert | | 15,92 | 20,95 | 39,70 | | | | |
| Gemessene IgG in ng/ml | 0,063 | 12,28 | 15,86 | 29,91 | 71,54% | 11,897 | 0,998 | 0,08 |
| Sollwert | | 11,9 | 18,53, | 37,28 | | | | |
| **Serum 6** | | | | | | | | |
| Gemessene IgG in ng/ml | 0,000 | 4,08 | 9,63 | 27,98 | 96,14% | 3,880 | 1,000 | |
| Sollwert | | 3,88 | 10,33 | 29,08 | | | | |
| Gemessene IgG in ng/ml | 0,125 | 3,39 | 8,00 | 16,52 | 50,89% | 4,002 | 0,988 | 0,18 |
| Sollwert | | 4,00 | 10,64 | 29,39 | | | | |
| **Serum 7** | | | | | | | | |
| Gemessene IgG in ng/ml | 0,000 | 3,67 | 7,36 | 26,82 | 95,20% | 2,700 | 0,991 | |
| Sollwert | | 2,70 | 9,92 | 28,67 | | | | |
| Gemessene IgG in ng/ml | 0,250 | 2,97 | 5,36 | 12,94 | 40,00% | 2,924 | 1,000 | 0,27 |
| Sollwert | | 2,94 | 9,22 | 27,97 | | | | |

Erläuterungen:

In dem Assay wurden 4 anonyme Seren von gesunden Blutspendepatienten verwendet. Alle Seren wurden mit bekannten IgE-Konzentrationen 6,25 und 25 ng/ml aufgestockt und vermessen. Bei den gleichen Seren wurden unterschiedliche E-25-Konzentrationen zugefügt, um so Therapieantikörper-haltige Seren zu simulieren. Die Berechnung der jeweiligen IgE-Konzentrationen erfolgt aus den Messwerten mit Hilfe der Umkehrfunktion der für die Standardkurve gültigen Auswertefunktion (In(IgE)= (5,39079+Logit(E))/0,8984).

Im Einzelnen zeigten sich folgende Ergebnisse:

1. Serum 3:

   Serum 3 war mit 6,25 und 25 ng/ml IgE aufgestockt worden. Ohne Zugabe von E-25 lag die Wiederfindung der zugegebenen IgE-Konzentrationen bei einem Ausgangswert des Serums von 12 ng/ml (1:10 verdünnt) bei 108 %,. Diese wich von einer erwarteten Wiederfindung von 100 % nicht signifikant ab.
   Der Zusatz von 0,031 µg/ml E-25 (Xolair) bewirkte keine signifikante Änderung der Wiederfindung der IgE-Konzentrationen.

2. Serum 5:

   Serum 5 war mit 6,25 und 25 ng/ml IgE aufgestockt worden. Ohne Zugabe von E-25 lag die Wiederfindung der zugegebenen IgE-Konzentrationen bei einem Ausgangswert des Serums von 15,9 ng/ml (1:10 verdünnt) bei 102 %,. Diese wich von einer erwarteten Wiederfindung von 100 % nicht signifikant ab.
   Der Zusatz von 0,063 µg/ml E-25 (Xolair) bewirkte eine signifikante Erniedrigung der Wiederfindung der IgE-Konzentrationen auf 72 %. Aus der o.g. aus den Standardkurven gewonnenen Beziehung (Formel 8) konnte somit auf eine E-25-Konzentration von 0,08 µg/ml geschlossen werden, die in der Nähe der tatsächlich zugegebenen E-25-Konzentration von 0,063 µg/ml lag.

3. Serum 6:

   Serum 6 war mit 6,25 und 25 ng/ml IgE aufgestockt worden. Ohne Zugabe von E-25 lag die Wiederfindung der zugegebenen IgE-Konzentrationen bei einem Ausgangswert des Serums von 3,9 ng/ml (1:10 verdünnt) bei 96 %,. Diese wich von einer erwarteten Wiederfindung von 100 % nicht signifikant ab.
   Der Zusatz von 0,125 µg/ml E-25 (Xolair) bewirkte eine signifikante Erniedrigung der Wiederfindung der IgE-Konzentrationen auf 50 %. Aus der o.g. aus den Standardkurven gewonnenen Beziehung (Formel 8) konnte somit auf eine E-25-Konzentration von 0,18 µg/ml geschlossen werden, die in der Nähe der tatsächlich zugegebenen E-25-Konzentration von 0,125 µg/ml lag.

4. Serum 7:

   Serum 7 war mit 6,25 und 25 ng/ml IgE aufgestockt worden. Ohne Zugabe von E-25 lag die Wiederfindung der zugegebenen IgE-Konzentrationen bei einem Ausgangswert des Serums von 2,7 ng/ml (1:10 verdünnt) bei 95 %,. Diese wich von einer erwarteten Wiederfindung von 100 % nicht signifikant ab.
   Der Zusatz von 0,125 µg/ml E-25 (Xolair) bewirkte eine signifikante Erniedrigung, der Wiederfindung der IgE-Konzentrationen auf 40 %. Aus der o.g. aus den Standardkurven gewonnenen Beziehung (Formel 8) konnte somit auf eine E-25-Konzentration von 0,27 µg/ml geschlossen werden, die in der Nähe der tatsächlich zugegebenen E-25-Konzentration von 0,25 µg/ml lag.

Es stehen nur sehr aufwändige Verfahren zur Bestimmung des gesamten und freien IgE zur Verfügung. In der Publikation Hamilton R.G. et al. Sind 2 gesonderte Elisas zur Bestimmung erforderlich, wobei eine Bestimmung der wirksamen Antikörperkonzentration nicht erfolgt.
Mit Hilfe des erfindungsgemäßen Verfahrens ist eine Therapiekontrolle beispielsweise bei dem Einsatz von Omalizumab bei allergischem Asthma und anderen Autoimmunerkrankungen sehr einfach durchzuführen.

Literatur

Rodbard D., Ratford P.L., Cooper J. and Ross G.T. (1968). Statistical quality control of radioimmunoassays. J. Clin.

Endocrinol. Metab. 28, 352.
Azimzadeh A., Pellequer J.L. and Van Regenmortel M.H.V. (1992). Operational aspects of antibody affinity constants measured by liquid-phase and solid-phase assays. J. Molecular Recognition. 5, 9.
Goldberg M.E. and Djavadi-Ohaniance L. (1993). Methods for measurement of antibody/antigen affinity based on ELISA and RIA. Current Opinion in Immunology 5, 278.
Hamilton R.G., MarcotteG.V., Saini S.S.: Immunological methods for quantifying free and total serum Ige levels in allergy patients receiving omalizumab (Xolair) therapy. J. Immunol. Methods. 2005, 303 (1-2):81-91
**Europäischen Patentschrift** EP 0850416B1 vom 01.08.2001

## Patentansprüche

1. Verfahren zur gleichzeitigen immunochemischen Bestimmung eines Analyten und eines gegen den Analyten gerichteten Therapieantikörpers, mit einem Immunoassay, mit nachstehenden Komponenten

    - einen an einer Oberfläche gebundenen gegebenenfalls markierten Fängerantikörper,
    - einen markierten Therapieantikörper, oder ein markierter Antikörper, der das gleiche Bindungsepitop wie der Therapieantikörper aufweist,
    - ein Antigen, das mit dem Fängerantikörper und dem Therapieantikörper an verschiedenen Epitopen bindet und **dadurch** einen immunochemischen Sandwich bildet,
    - eine Lösung des unmarkierten Therapieantikörpers bekannter Konzentration und eine Antigenlösung zur Aufstockung der Proben,

    **dadurch gekennzeichnet, dass**

    - Sandwich-Immunoassay-Standardkurven bei 2-5 verschiedenen Konzentrationen der unmarkierten Therapieantikörper aufgestellt werden,
    - der Zusammenhang zwischen der Verringerung der rekalkulierten Antigenkonzentration (Standardkonzentration) durch Zusatz der Therapieantikörper mit Hilfe einer statistischen Auswertung erfasst wird,
    - Proben mit unbekannten Antigenkonzentrationen mit 2-3 bekannten Antigenkonzentrationen aufgestockt und die Wiederfindungen gemessen werden,
    - mit den ermittelten Wiederfindungswerten in den Proben anhand der der Wiederfindungswerte in den Eichkurven auf die Konzentration des Therapieantikörpers und auf die tatsächliche totale Konzentration des Antigens geschlossen wird.

## Claims

1. Method for simultaneous immunochemical determination of an analyte and a therapy antibody aimed against the analyte, with an immunoassay with the following components

    - a catcher antibody bound to a surface and marked if need be,
    - a marked therapy antibody or a marked antibody which manifests the same binding epitope as the therapy antibody,
    - an antigen, which binds with the catcher antibody and the therapy antibody at various epitopes and thus forms an immunochemical sandwich,
    - a solution of the unmarked therapy antibody of known concentration and an antigen solution to stock up the samples,

    wherein

    - sandwich-immunoassay standard curves are produced in 2-5 various concentrations of the unmarked therapy antibodies,
    - the connection between the reduction of the recalculated antigen concentration (standard concentration) is detected by addition of the therapy antibodies with the help of a statistical evaluation,
    - samples with unknown antigen concentrations are stocked up with 2-3 known antigen concentrations and the recoveries are measured,
    - the concentration of the therapy antibody and the actual total concentration of the antigen are concluded from the determined recovery figures in the samples on the basis of the recovery figures in the calibration curves.

## Revendications

1. Procédé permettant de déterminer simultanément au niveau immunochimique un analyte et un anticorps thérapeutique dirigé contre l'analyte, avec un assay d'immunologie, avec les composants ci-contre

   - un anticorps capteur lié à une surface, le cas échéant marqué,
   - un anticorps thérapeutique marqué ou un anticorps marqué qui présente le même épitope de liaison que l'anticorps thérapeutique,
   - un antigène qui se lie avec l'anticorps capteur et l'anticorps thérapeutique à différents épitopes pour former ainsi un sandwich immunochimique,
   - une solution de l'anticorps thérapeutique non marqué de concentration connue et une solution antigène pour augmenter les échantillons,

   **se caractérisant par** le fait

   - les courbes standard de l'assay immunologique sandwich sont établies pour 2 à 5 concentrations différentes des anticorps thérapeutiques non marqués,
   - le rapport entre la diminution de la concentration en anticorps recalculée (concentration standard) par addition des corps thérapeutiques est saisi à l'aide d'une exploitation statistique,
   - les échantillons ayant des concentrations en antigène inconnues sont augmentés par 2 à 3 concentrations d'antigène connues et la récupération est mesurée,
   - se conclut avec les valeurs de récupération déterminées dans les échantillons à l'aide des valeurs de récupération dans les courbes d'étalonnage sur la concentration de l'anticorps thérapeutique et sur la concentration totale réelle de l'antigène.

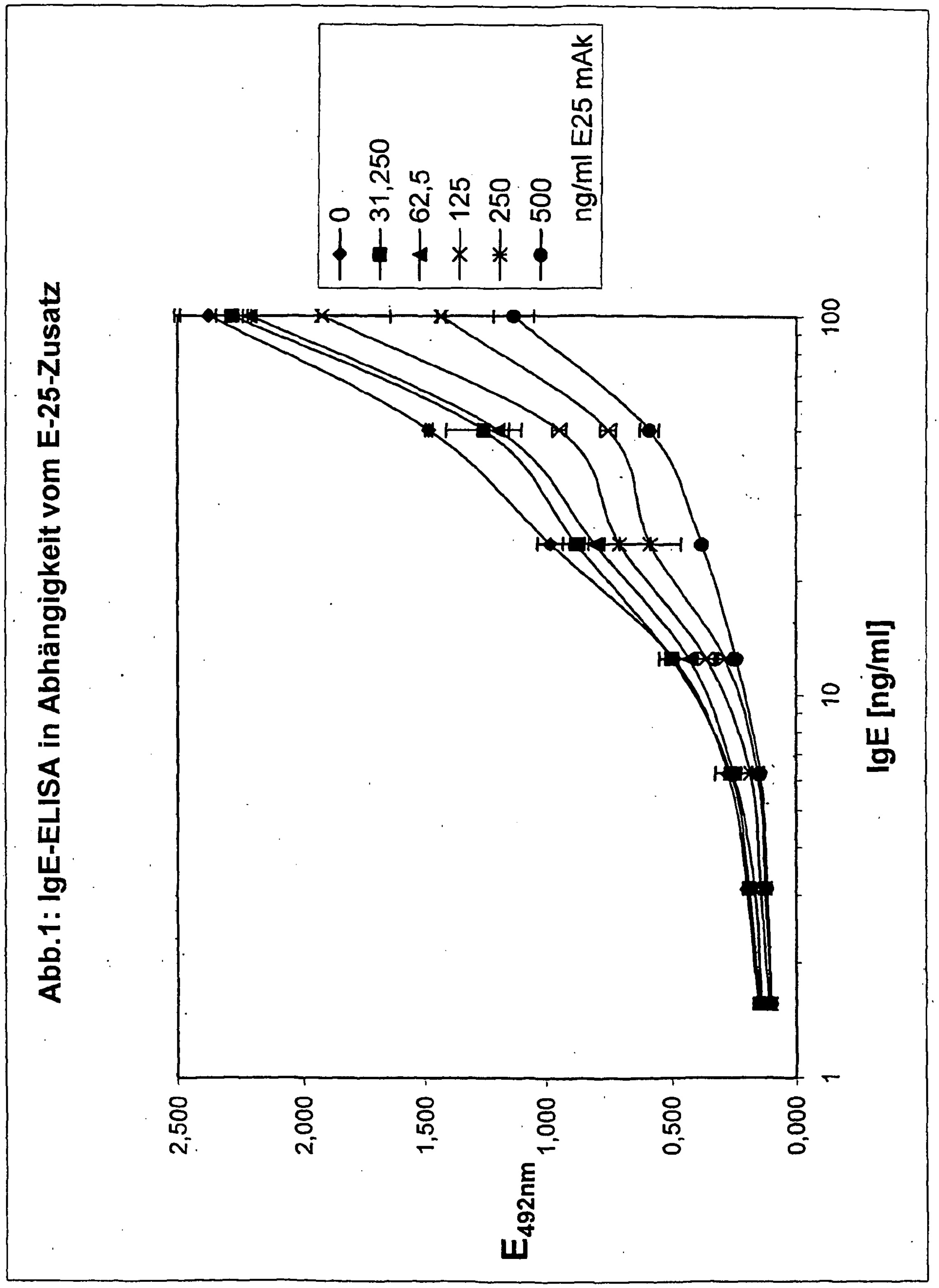
Abb.1: IgE-ELISA in Abhängigkeit vom E-25-Zusatz
E492nm
2,500
2,000
1,500
1,000
0,500
0,000
1
10
100
IgE [ng/ml]
0
31,250
62,5
125
250
500
ng/ml E25 mAk

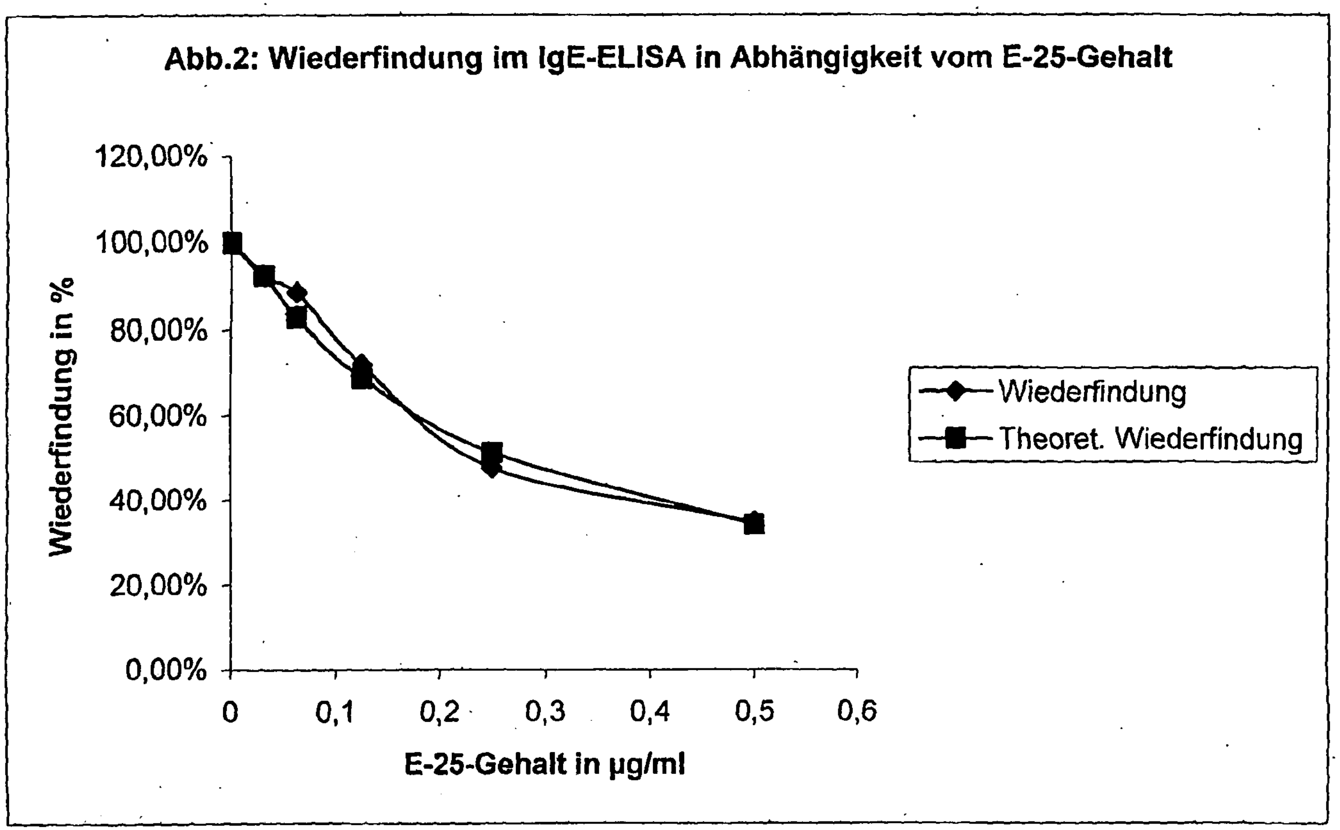

Abb.2: Wiederfindung im IgE-ELISA in Abhängigkeit vom E-25-Gehalt

1/WF = 0,9606 + 3,932*X (Formel 5)

WF: Wiederfindung

X: E-25-Gehalt in µg/ml

Das Bestimmtheitsmaß für diese Funktion war 98,74 %.

Abb. 3: IgE-ELISA mit und ohne Zusatz vor E-25

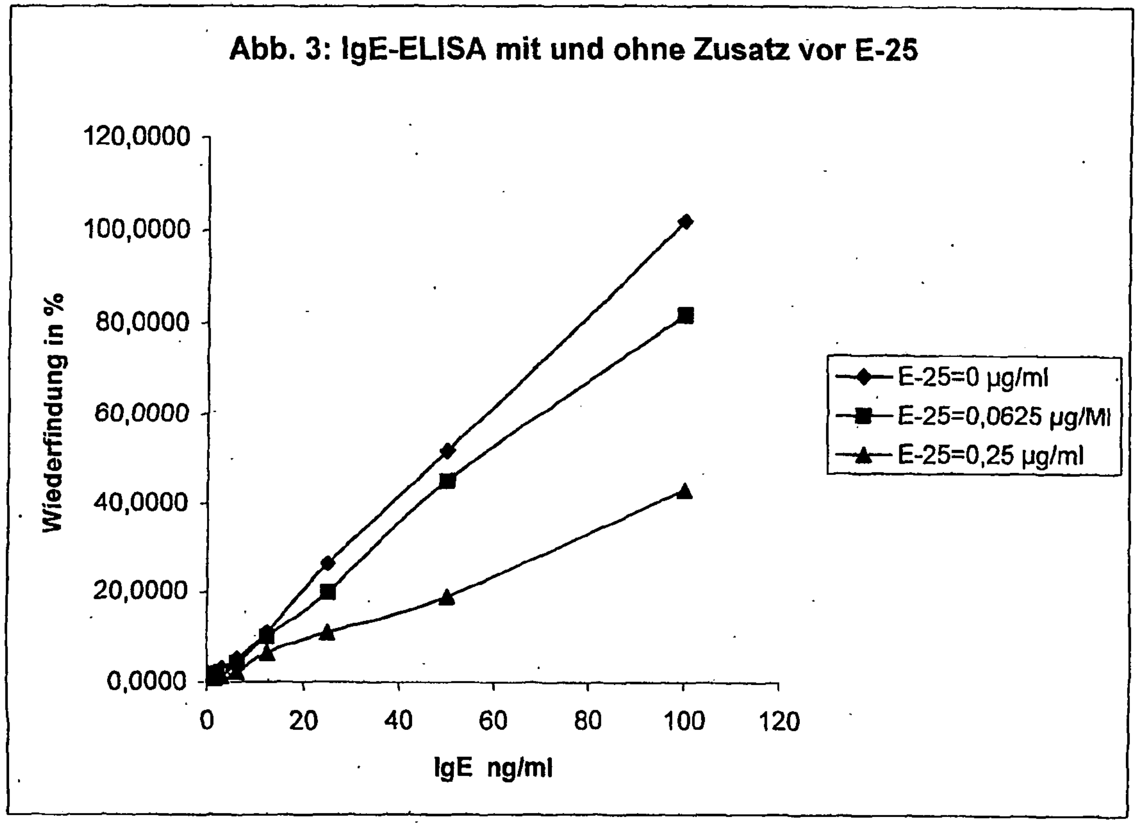


Wf = 0,99 – 2,291*X (Formel 8)
Wf: Wiederfindung der IgE-Konzentration
X: E-25-Zusatz in µg/ml
Das Bestimmtheitsmaß für diesen linearen Zusammenhang war 99,84 %.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0850416 B1


### In der Beschreibung aufgeführte Nicht-Patentliteratur


- **Rodbard D. ; Ratford P.L. ; Cooper J. ; Ross G.T.** Statistical quality control of radioimmunoassays. *J. Clin. Endocrinol. Metab.,* 1968, vol. 28, 352
- **Azimzadeh A. ; Pellequer J.L. ; Van Regenmortel M.H.V.** Operational aspects of antibody affinity constants measured by liquid-phase and solid-phase assays. *J. Molecular Recognition.,* 1992, vol. 5, 9
- **Goldberg M.E. ; Djavadi-Ohaniance L.** Methods for measurement of antibody/antigen affinity based on ELISA and RIA. *Current Opinion in Immunology,* 1993, vol. 5, 278
- **Hamilton R.G. ; MarcotteG.V. ; Saini S.S.** Immunological methods for quantifying free and total serum Ige levels in allergy patients receiving omalizumab (Xolair) therapy. *J. Immunol. Methods.,* 2005, vol. 303 (1-2), 81-91